# FASCICULE DE BREVET EUROPEEN

(11) **EP 4 415 534 B1**
(45) Date de publication et mention de la délivrance du brevet: **10.09.2025**
(21) Numéro de dépôt: 22793241.5
(22) Date de dépôt: 13.10.2022
(51) Int. Cl.: A01K 67/30

(54) **SYSTEME DE MANUTENTION DE BACS DE PRODUCTION D'INSECTES**
SYSTEM ZUR HANDHABUNG VON INSEKTENPRODUKTIONSSCHALEN
SYSTEM FOR HANDLING INSECT PRODUCTION TRAYS

(30) Priorité: 21.11.2021 FR 2112002
(43) Date de publication de la demande: 21.08.2024
(73) Titulaire: Invers, 63720 Saint-Ignat (FR)
(72) Inventeur: CREPIEUX, Sébastien, 63720 Saint-Ignat (FR)
(74) Mandataire: Gabriel, Franck
(86) Numéro de dépôt international: PCT/IB2022/059802
(87) Numéro de publication internationale: WO 2023/084338

(56) Documents cités:
- WO-A1-2014/171829
- CN-A- 113 519 463
- KR-B1- 101 432 560
- TW-A- 202 121 981

## Description

### DOMAINE TECHNIQUE DE L'INVENTION

La présente invention concerne un système de manutention de bacs de production d'insectes, pour une installation de production d'insectes, notamment pour l'alimentation animale.

### ETAT DE LA TECHNIQUE ANTERIEURE

Diverses approches existent pour mettre en œuvre l'élevage d'insectes à grande échelle. Les premières méthodes étaient entièrement manuelles, fastidieuses et avec des taux de productivité peu élevés. Peu à peu, la technicité des équipements utilisés a permis de réduire les interventions humaines dans le processus et d'augmenter la productivité. Aujourd'hui, de nombreux systèmes avec divers équipements technologiques existent.

La production d'insectes à échelle industrielle implique en général deux cycles ou phases complémentaires, soit un premier pour obtenir des œufs, et un second pour permettre la croissance des larves ainsi obtenues. Ces deux cycles ayant chacun leurs spécificités sont aujourd'hui réalisés de façon totalement indépendante, avec des moyens spécifiques à chacun.

Par exemple, le document WO2016153340 décrit un système et un procédé pour effectuer la reproduction des insectes à l'aide de reproducteurs produisant des œufs. Le système est particulièrement adapté pour les cycles de reproduction et ne convient pas pour effectuer des cycles de production d'insectes à partir des œufs. Pour effecteur les deux cycles, une installation industrielle doit donc prévoir deux types de systèmes différents, soit un pour produire les œufs, et un pour produire les larves et/ou insectes. Une telle installation requière une grande surface, présente une double complexité, et occasionne un surcoût important.

Le document FR3034622 décrit un atelier d'élevage d'insectes, comportant une première zone dans laquelle les insectes en cours d'élevage sont stockés au cours de leur croissance dans des contenants et une seconde zone comportant au moins un poste configuré pour la réalisation d'une opération d'élevage sur les insectes d'un contenant ou sur ledit contenant. Les contenants sont groupés dans la première zone en ensembles de contenants palettisés dits unités élémentaires. La première zone comporte des rayonnages à palettes dans lesquels sont disposées les unités élémentaires. La première zone est en outre équipée d'un dispositif automatisé configuré pour le déplacement des unités élémentaires entre la première zone et une interface avec la seconde zone. L'atelier prévoit, dans la seconde zone, une pluralité de postes spécialisés chacun conçu pour effectuer une opération spécifique. Il en résulte une installation complexe, coûteuse, requérant une très grande surface.

Le document FR3106252 décrit un système de manutention de bacs agencé sous forme d'un îlot de production, les différents éléments étant agencés les uns à côtés des autres. L'agencement décrit comporte un bras manipulateur qui agrippe les bacs et les déplace à différents endroits de l'îlot en fonction de l'opération à effectuer. Les déplacements à effectuer sont parfois complexes. Un tel bras est très coûteux et opère de façon relativement lente avec un seul bac à la fois. Par ailleurs, le moyen de vidage et le moyen de remplissage sont séparés et les étapes de vidage et de remplissage sont disjointes. Il en résulte un cycle global long et une implantation sur une surface importante.

Le document US2019/0387704 décrit un système de manutention de bacs permettant de récupérer des matières de fin de cycle de production.

Le document CN113519463 décrit un système selon le préambule de la revendication 1 et un système d'élevage d'insectes dans lequel des bacs d'élevage sont remplis de nourriture et d'œufs ou larves et sont ensuite empilés et stockés en plusieurs piles agencées sur un support, les unes à côté des autres. Les bacs empilés sont conservés sur le support pendant quelques jours, correspondant à la période d'élevage. Les bacs sont ensuite récupérés un à un, vidés, et le contenu des bacs est trié dans une trieuse afin de séparer les insectes des autres matières. Pour manipuler les bacs, effectuer le remplissage et la mise en place pour stockage ainsi que pour vider les bacs, le système comporte un manipulateur, un élément de remplissage, une plate-forme de levage des bacs, un élément pour retourner et vider les bacs et une trieuse. Tous ces éléments du système d'élevage sont agencés en hauteur, le remplissage étant en position supérieure, la trieuse en position basse, le manipulateur et l'élément de retournement étant en position intermédiaire. Par ailleurs, les bacs empilés sont toujours stockés dans la zone de stockage, placée d'un côté du système d'élevage, qui sert en même temps de zone d'élevage. Les éléments permettant de manipuler les bacs sont agencés de l'autre côté du système d'élevage.

Pour pallier ces différents inconvénients, l'invention prévoit différents moyens techniques.

### EXPOSE DE L'INVENTION

Tout d'abord, un premier objectif de l'invention consiste à fournir un système permettant de traiter de façon rapide et fiable des lots de bacs en fin de cycle de production et de préparer les lots suivants pour les prochains cycles de production.

Pour ce faire, l'invention prévoit un système de manutention pour mise en oeuvre d'une pluralité d'opérations de transition entre deux cycles de production avec une pluralité de bacs en fin d'un cycle d'élevage d'insectes dans lesdits bacs, et préparation de ces bacs pour un cycle suivant d'élevage d'insectes, comprenant :
i) un portique de réception d'une pile de bacs en fin de cycle d'élevage;
ii) un portique de palettisation d'une pile de bacs pour amorce d'un prochain cycle de production d'insectes;
iii) une enceinte de manutention conçue pour mettre en oeuvre l'ensemble des opérations de transition des bacs entre les deux portiques ;
l'enceinte de manutention comportant une tour de manutention des bacs dans laquelle sont agencés verticalement :
iv) un module de manipulation de bacs, pour déplacer les bacs en translation et en rotation ;
v) un module de réception des matières de fin de cycles de production agencé sous le module de manipulation de bacs ;
vi) un module de remplissage avec matière d'amorce d'un prochain cycle de production d'insectes, agencé au-dessus du module de manutention de bacs, caractérisé en ce que dans le système (1) de manutention, le portique de réception d'une pile entrante de bacs d'une fin de cycle de production et le portique de palettisation d'une pile sortante d'amorce d'un prochain cycle de production d'insectes sont agencées de chaque côté de la tour de manutention.

Le système comporte les équipements nécessaires pour effectuer la manutention de bacs issus d'une fin de cycle de production, en particulier pour saisir au moins un bac, effectuer un vidage du ou des bacs pour permettre de récupérer la matière de fin de cycle de production, remplir le ou les bacs de matière d'amorce d'un nouveau cycle de production d'insectes et effectuer une mise en place des bacs remplis en pile. L'utilisation d'une tour telle que décrit permet réaliser l'ensemble de ces opérations en une seule et unique phase. Chaque phase regroupe l'ensemble des opérations effectuées entre la prise d'un ou plusieurs bacs remplis de matière de fin de cycle de production dans un portique de réception et la mise en place du ou des bacs remplis de matière d'amorce de cycle de production dans un portique de palettisation.

Cet agencement permet de réaliser l'ensemble des manipulations à l'aide d'un seul et unique outil de manipulation, contribuant à minimiser l'espace requis et à réduire les coûts du système. Cet outil de manipulation est multi-phases afin de réaliser les opérations pour les deux phases en cours, à savoir une phase de récupération de matière de production d'un cycle de production achevé et une phase de préparation de bacs avec ajout de matière d'amorce d'un nouveau cycle de production.

Cet agencement avec une tour entourée par les deux portiques permet de minimiser les déplacements. Le système occupe un emplacement restreint et les temps de cycles sont réduits au minimum.

Selon un mode de réalisation avantageux, le module de manipulation de bacs comporte des rails agencés entre le portique de réception et le portique de palettisation, un chariot mobile étant monté sur lesdits rails.

Cette architecture est simple, avec un seul élément mobile effectuant l'ensemble des opérations sur les bacs.

De manière avantageuse, le chariot mobile comporte un pivot adapté pour incliner au moins un bac et verser son contenu.

Egalement de manière avantageuse, le module de réception des matières de fin de cycles de production comprend un tamiseur-récupérateur pour matière de fin de cycle conçu pour séparer les constituants à récupérer de la matière de fin de cycle de production et acheminer ces constituants vers au moins un point de récupération des matières séparées.

Cette architecture permet de séparer les constituants de la matière de fin de cycle de production en temps réel, lors du déversement du contenu des bacs dans la tour de manutention.

Le tamiseur-récupérateur comporte avantageusement au moins deux niveaux de tamisage. Les multiples niveaux de tamisage permettent de trier plusieurs éléments constituants en fonction de leur granulométrie.

Le tamiseur est un organe efficace et performant pour effectuer les opérations de sélection ou tri des constituants du contenu des bacs, même pour des bacs de grande dimension. Le tamiseur permet aisément de traiter tout le contenu d'un bac entier voire de plusieurs bacs. La superposition des étages, avec des tamis de granulométrie différente, permet d'effectuer sur une surface contenue plusieurs opérations de séparation avec acheminement vers des points de récupération des constituants.

De manière avantageuse, la récupération des constituants pour un cycle de ponte et la récupération des constituants pour un cycle de production de larves ou d'insectes peuvent être effectuée dans le même tamiseur-récupérateur. Le tamiseur-récupérateur multi-cycles permet d'adapter le système de manutention de façon simple et rapide, à des coûts réduits, et dans un emplacement restreint.

Selon un autre mode de réalisation avantageux, le système de manutention comprend un système d'aspiration agencé pour récupérer les mues des larves et les poussières volatiles. Les manipulations effectuées avec les bacs, en particulier l'étape de déversement du contenu des bacs dans la trémie, engendrent d'importantes quantités de poussières qui peuvent rester en suspension dans l'air un certain temps. Leur récupération permet d'assainir l'air ambiant. Ce moyen simple et compact permet par ailleurs de récupérer les mues de larves. On utilise par exemple un anneau de Pouyès. Le système permet avantageusement d'assurer une bonne qualité de l'air ambiant.

De manière avantageuse, le portique de réception de bacs et le portique de palettisation de bacs comportent un élévateur conçu pour permettre le positionnement d'un bac situé sur le dessus d'une pile de bacs au niveau du module de manipulation.

Un procédé de manutention est également prévu pour mise en oeuvre d'une pluralité d'opérations de transition entre deux cycles de production avec une pluralité de bacs en fin d'un cycle d'élevage d'insectes dans lesdits bacs, et préparation de ces bacs pour un cycle suivant d'élevage d'insectes, comprenant les étapes suivantes :
i) réception d'une pluralité de bacs entrant en fin de cycle d'élevage d'insectes pour récupération du contenu ;
ii) saisie d'un bac entrant dans un portique de réception logeant une pile de bacs de production d'insectes par un module de manipulation en translation et rotation de bacs pour translation de ce bac depuis la pile de bacs entrant vers une tour de traitement ;
iii) retournement du bac saisi par le module de manipulation et récupération du contenu par un module de réception ;
iv) retournement du bac par le module de manipulation pour réception de matière nouvelle ;
v) remplissage du bac avec matière d'amorce d'un prochain cycle de production d'insectes par un module de remplissage ;
vi) translation du bac nouvellement rempli pour mise en place dans un portique de palettisation en une pile de bacs sortant successivement remplis ;
vii) mise en oeuvre successive des étapes ii à vi jusqu'à dépalettisation complète de la pile de bacs entrant et palettisation complète d'une pile de bacs sortant.

Ce procédé permet d'effectuer automatiquement de façon simple, efficace et rapide, l'ensemble des opérations de manipulations des bacs de production en fin de cycle de croissance des larves afin notamment de récupérer ces dernières, et en début de cycle de production de larves, afin de charger la matière d'amorce de production requise dans chacun des bacs.

De manière avantageuse, les bacs effectuant la transition entre les deux portiques sont les mêmes. On évite ainsi d'une part de stocker les bacs arrivant de la production et d'autre part de devoir utiliser un stock de nouveaux bacs pour amorcer les cycles de production suivants.

Selon un mode de réalisation avantageux, les phases de récupération de matières de fin de cycle de production et de préparation de bacs avec matières d'amorce de production ultérieure sont imbriquées de façon à ce que une pile de bacs d'amorce de cycle constituée corresponde à une pile de bacs de fin de cycle dépilée.

De manière avantageuse, le procédé est utilisé pour produire des coléoptères, plus préférentiellement des tenebrio, et encore plus préférentiellement des tenebrio molitor. Ces types d'insectes sont particulièrement bien adaptés à ce type de procédé. Ils comportent par ailleurs de grandes qualités nutritives, dont un taux élevé de protéines.

De manière avantageuse, les insectes produits sont au stade larvaire. En effet, c'est le stade de croissance permettant l'obtention d'un taux élevé de protéines, tout en conservant des cycles de production relativement courts.

On utilise avantageusement des bacs de dimensions relativement imposantes, par exemple de 800 cm par 600 cm. Une fois chargés, les bacs sont trop lourds pour pouvoir être manipulés manuellement par un opérateur lors des phases de fonctionnement à échelle industrielle, impliquant de manipuler plusieurs bacs par minute. Le système prévu permet d'effectuer ces opérations sans affecter la sécurité des opérateurs, qui peuvent ainsi se concentrer sur les opérations de contrôle et surveillance.

Selon un mode de réalisation avantageux, les bacs sont empilables et comportent des plots de guidage facilitant leur empilage et pouvant par ailleurs servir de cible pour le système de localisation et guidage.

### DESCRIPTION DES FIGURES

Tous les détails de réalisation sont donnés dans la description qui suit, complétée par les figures 1 à 4, présentées uniquement à des fins d'exemples non limitatifs, et dans lesquelles :
- la figure 1 est une représentation schématique en élévation d'un exemple de réalisation d'un système de manutention de bacs ;
- la figure 2 est une vue en perspective d'un exemple de module de manipulation de bacs ;
- la figure 3 montre, en perspective, le tamiseur-récupérateur du système de la figure 1 ;
- la figure 4 est une vue de face d'un exemple de système de manutention de bacs.

### DESCRIPTION DETAILLEE DE L'INVENTION

### DEFINITIONS

Par *« matières de fin de cycle de production »,* on entend des larves, déjections, résidus de mues, restes de nourriture pour un cycle d'élevage de larves ou insectes, et/ou de reproducteurs, oeufs, restes de nourriture et déjections pour un cycle de ponte.

Par *« matières d'amorce de cycle de production »,* on entend de la nourriture avec oeufs, ou nourriture avec insectes reproducteur, selon le cycle.

Par *« substrat »,* on entend une matière constituant le milieu de vie des oeufs, larves, insectes ou reproducteur.

Par « *substrat* résiduel », on entend le substrat présent en fin de cycle de production.

Par « produit final », on entend les larves en fin de croissance ou des insectes comme par exemple des coléoptères adultes.

### SYSTEME DE MANUTENTION

Pour produire de grandes quantités d'insectes ou larves, on met en place des fermes ou usines d'insectes. Une ferme comprend par exemple un ou plusieurs emplacements de stockage pour placer une pluralité de piles de bacs. Un ou plusieurs dispositifs complémentaires peuvent être prévus par exemple pour ajouter des doses d'arrosage lorsque nécessaire ou à des intervalles préétablis.

Le système de manipulation préalablement décrit peut être agencé dans une zone réservée de l'emplacement de stockage, ou dans une pièce ou local avoisinant.

Après leur croissance, les larves ou insectes peuvent subir une ou plusieurs étapes de transformation en produit alimentaire. On prévoit par exemple une phase de déshydratation, afin que le produit alimentaire obtenu soit facile à conserver et à manutentionner.

La technologie présentée consiste en un système et procédé permettant d'effectuer de façon efficace et optimale la transition entre une fin de cycle d'élevage ou production d'insectes et l'amorce d'un nouveau cycle d'élevage ou production d'insectes, en utilisant les mêmes bacs.

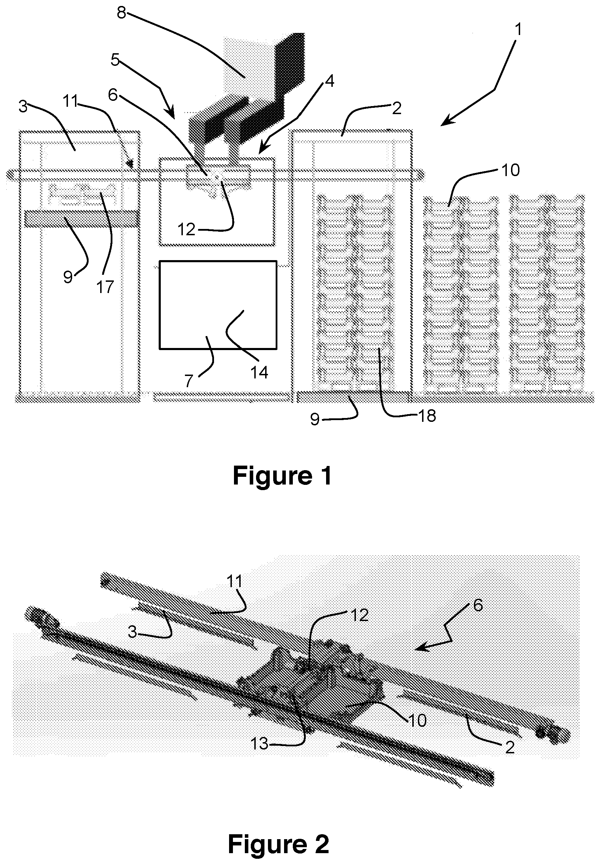

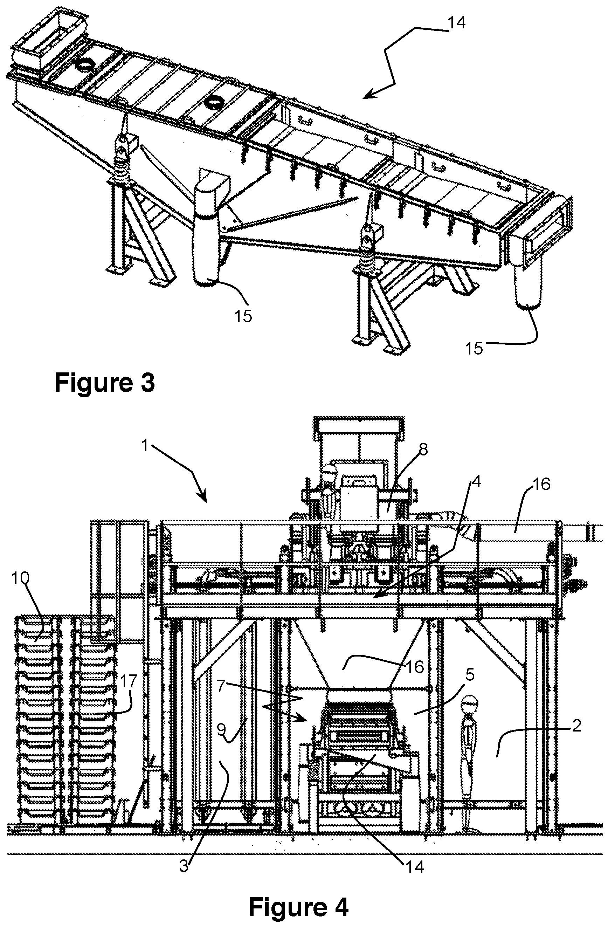

Ces bacs 10 servent à loger les larves en cours de croissance ainsi que les nutriments utilisés pour assurer cette croissance. Au cours de leur croissance, les larves produisent des déjections qui vont également être contenus dans les bacs. En pratique, pour utiliser un espace le plus restreint possible, les bacs 10 sont empilés les uns sur les autres, tel que montré dans les exemples des figures 1 et 4, pour former des piles de bacs pouvant comprendre cinq à dix bacs ou quinze bacs, voire plus.

Pour permettre d'empiler les bacs les uns par dessus les autres, chaque bac comporte des éléments de support conçus pour permettre de positionner deux bacs identiques l'un dans l'autre en laissant des fenêtres latérales au moins partiellement libres, à des fins d'aération.

Tel que montré à la figure 1, complétée par l'exemple de la figure 4, le système 1 de manutention comporte deux piles de bacs 10, soit une pile 18 de bacs entrant et une pile 17 de bacs sortant pour un nouveau cycle. Un portique de réception 2 permet de loger et dépalettiser la pile 18 de bacs en fin de cycle d'élevage. Un portique de palettisation 3 permet de constituer et loger une pile 17 de bacs d'amorce d'un prochain cycle de production d'insectes.

Entre les deux portiques, une enceinte 4 de manutention est conçue pour mettre en oeuvre l'ensemble des opérations de transition des bacs 10 entre deux cycles de production.

Dans l'exemple illustré, l'enceinte de manutention prend la forme d'une tour 5 dans laquelle les différents modules de travail sont agencés les uns au-dessus des autres.

Tout d'abord, on retrouve un module 6 de manipulation de bacs, prévu, d'une part pour déplacer les bacs en translation entre les deux portiques, et d'autre part pour permettre aux bacs de pivoter lorsqu'ils sont dans la tour pour déverser le contenu des bacs et revenir à l'horizontal afin que les bacs puissent être remplis. Sous le module de manipulation 6 de bacs, un module 7 de réception des matières de fin de cycles de production pour recevoir les matières déversées par les bacs lorsqu'ils sont retournés, est disposé dans la tour. Dans l'exemple illustré, le module 7 de réception des matières de fin de cycle de production comprend un tamiseur-récupérateur 14 pour séparer les constituants à récupérer de la matière de fin de cycle de production et acheminer ces constituants vers au moins un point 15 de récupération des matières séparées, tel que montré dans l'exemple de la figure 3. Le tamiseur-récupérateur 14 comporte de préférence au moins deux niveaux de tamisage. Chaque étage comporte un tamis de granulométrie spécifique, dont les dimensions des orifices sont prévues en fonction des dimensions moyennes des constituants à récupérer. Le tamiseur-récupérateur est de préférence conçu de façon à ce que la récupération des constituants issus d'un cycle de ponte et la récupération des constituants issus d'un cycle d'élevage de larves ou d'insectes puisse être effectuée dans le même tamiseur-récupérateur, permettant ainsi de prévoir un unique tamiseur-récupérateur et/ou un unique système de manutention pour le procédé correspondant de mise en oeuvre.

Au-dessus du module 6 de manipulation de bacs, un module de remplissage 8 avec matière d'amorce d'un prochain cycle de production d'insectes, est disposé dans ou sur la tour. Ce module permet d'acheminer la matière requise par exemple à l'aide d'une vis sans fin ou d'un convoyeur ou autre moyen d'acheminement adapté.

Tel que montré à la figure 2, pour assurer le déplacement des bacs en translation entre les deux portiques, le module 6 de manipulation de bacs comporte des rails 11 agencés entre le portique 2 de réception et le portique 3 de palettisation. Un chariot 12 mobile est monté sur ces rails 11. Chaque portique comprend un élévateur 9 conçu pour permettre le positionnement d'un bac situé sur le dessus d'une pile de bacs au niveau du module 6 de manipulation, par exemple en alignement entre les rails 11, afin que le chariot puisse saisir ce bac, par exemple à l'aide de pinces, mâchoires, ou crochets, ou tout autre moyen de préhension adapté.

Un ou plusieurs pivots 13 sont agencés sur le chariot mobile et adaptés pour faire pivoter les bacs et verser leur contenu dans le module 7 de réception des matières de fin de cycle de production.

La manipulation des bacs, en particulier le déversement des matières de fin de cycle de production, est susceptible d'engendrer la formation de poussières pouvant se répandre dans l'atmosphère ambiant. Un système d'aspiration 16 (montré dans l'exemple de la figure 4) agencé dans la tour 5, permet de récupérer ces poussières. Les poussières peuvent comprendre les mues des larves et diverses poussières issues de la production des insectes.

Dans l'exemple illustré à la figure 1, deux piles de bacs sont agencées côte à côte et le portique 2 de réception est adapté pour loger ces deux piles. Le chariot mobile est également conçu pour loger, transporter et manipuler deux bacs en même temps. En variante, le système et le procédé de manutention sont conçus pour une seule pile de bacs, ou pour quatre piles de bacs, chacun des éléments du système de manutention étant adaptés en fonction de ce nombre.

### PROCEDE

Le système préalablement décrit permet la mise en œuvre d'un procédé selon deux déclinaisons, soit une première déclinaison pour assurer la transition entre deux cycles de production d'insectes ou larves (ou déclinaison de production du produit final), et une seconde déclinaison pour assurer la transition entre deux cycles de production d'œufs d'insectes (ou déclinaison de reproduction).

Dans chaque cas, le procédé comporte deux phases, permettant de faire la jonction entre deux cycles successifs de production. On retrouve ainsi une phase de récupération de matières de production provenant d'un cycle antérieur de production de larves ou d'insectes ou d'œufs et une phase de préparation de bacs pour un cycle ultérieur de production de larves ou d'insectes ou d'œufs visant à préparer une pile d'amorce de cycle.

Selon la configuration du système de manutention, une ou plusieurs piles de bacs 10 contenant les matières inhérentes à une fin de cycle d'élevage sont reçues et disposées dans un portique de réception 2. L'élévateur 9 soulève les piles de bacs de façon à aligner les bacs du haut avec les moyens de préhension du chariot 12 mobile. Les bacs alignés sont agrippés par les moyens de préhension du chariot mobile. Le chariot mobile peut ensuite déplacer les bacs jusqu'à l'intérieur de la tour 5. Une fois en place au-dessus du module 7 de réception de matière de fin de cycle de production, les bacs sont pivotés ou inclinés afin de déverser leur contenu. Les matières reçues par le tamiseur-récupérateur 14 s'écoulent dans les différents tamis et sont séparées puis récupérées pour traitement ultérieur.

Une fois vidés de leur contenu, les bacs sont remis à l'horizontal pour être remplis de matière d'amorce d'un prochain cycle de production à l'aide du module 8 de remplissage. Après remplissage, les bacs sont transportés par le chariot mobile vers le portique 3 de palettisation.

Le cycle peut alors reprendre avec la saisie des prochains bacs des piles de bacs entrant, jusqu'à épuisement de ces piles. Au début de la phase d'empilement des bacs, les premiers bacs remplis sont posés sur l'élévateur 9 du portique de palettisation. En fonction de l'arrivée des bacs suivants, l'élévateur descend et libère l'emplacement du dessus de la pile pour permettre la mise en place des bacs successifs, jusqu'à constitution des piles complètes, qui peuvent alors être retirées du portique de palettisation.

### Numéros de référence employés sur les figures

| | |
|---|---|
| 1 | Système de manutention de bacs de production d'insectes |
| 2 | Portique de réception de bacs |
| 3 | Portique de palettisation |
| 4 | Enceinte de manutention |
| 5 | Tour de manutention |
| 6 | Module de manipulation des bacs (en translation et rotation) |
| 7 | Module de réception des matières |
| 8 | Module de remplissage |
| 9 | Elévateur |
| 10 | Bacs |
| 11 | Rails |
| 12 | Chariot mobile (préférence avec capacité de deux bacs) |
| 13 | Pivot |
| 14 | Tamiseur-récupérateur |
| 15 | Point de récupération des matières séparées |
| 16 | Système d'aspiration |
| 17 | Pile de bacs d'amorce de cycle |
| 18 | Pile de bacs de fin de cycle |

## Revendications

1. Système (1) de manutention pour mise en œuvre d'une pluralité d'opérations de transition entre deux cycles de production avec une pluralité de bacs (10) en fin d'un cycle d'élevage d'insectes dans lesdits bacs, et préparation de ces bacs pour un cycle suivant d'élevage d'insectes, comprenant :
i) un portique de réception (2) d'une pile (18) de bacs en fin de cycle d'élevage;
ii) un portique de palettisation (3) d'une pile (17) de bacs pour amorce d'un prochain cycle de production d'insectes;
iii) une enceinte (4) de manutention conçue pour mettre en œuvre l'ensemble des opérations de transition des bacs entre les deux portiques ;
dans lequel l'enceinte (4) de manutention comporte une tour (5) de manutention des bacs dans laquelle sont agencés verticalement :
iv) un module (6) de manipulation de bacs, pour déplacer les bacs en translation et en rotation ;
v) un module (7) de réception des matières de fin de cycles de production agencé sous le module de manipulation (6) de bacs ;
vi) un module de remplissage (8) avec matière d'amorce d'un prochain cycle de production d'insectes, agencé au-dessus du module (6) de manutention de bacs, **caractérisé en ce que** dans le système (1) de manutention, le portique de réception (2) d'une pile entrante de bacs d'une fin de cycle de production et le portique de palettisation (3) d'une pile sortante d'amorce d'un prochain cycle de production d'insectes sont agencées de chaque côté de la tour (5) de manutention.

2. Système (1) de manutention selon la revendication 1, dans lequel le module (6) de manipulation de bacs comporte des rails (11) agencés entre le portique (2) de réception et le portique (3) de palettisation, un chariot (12) mobile étant monté sur lesdits rails (11).

3. Système (1) de manutention selon la revendication 2, dans lequel le chariot (12) mobile comporte un pivot (13) adapté pour incliner au moins un bac et verser son contenu.

4. Système (1) de manutention selon la revendication 1, dans lequel le module (7) de réception des matières de fin de cycles de production comprend un tamiseur-récupérateur (14) pour matière de fin de cycle conçu pour séparer les constituants à récupérer de la matière de fin de cycle de production et acheminer ces constituants vers au moins un point (15) de récupération des matières séparées.

5. Système de manutention selon la revendication 4, dans lequel le tamiseur-récupérateur (14) comporte au moins deux niveaux de tamisage.

6. Système de manutention selon l'une quelconque des revendications 1 à 5, comprenant un système d'aspiration (16) agencé pour récupérer les mues des larves et les poussières issues de la production des insectes.

7. Système de manutention selon l'une quelconque des revendications 1 à 6, dans lequel le portique (2) de réception de bacs et le portique (3) de palettisation de bacs comportent un élévateur (9) conçu pour permettre le positionnement d'un bac situé sur le dessus d'une pile de bacs au niveau du module (6) de manipulation.

## Patentansprüche

1. Handhabungssystem (1) zum Durchführen einer Vielzahl von Übergangsoperationen zwischen zwei Produktionszyklen mit einer Vielzahl von Behältern (10) am Ende eines Insektenzuchtzyklus in den genannten Behältern und zum Vorbereiten dieser Behälter für einen nachfolgenden Insektenzuchtzyklus, umfassend:
i) einen Empfangsportal (2) für einen Stapel (18) von Behältern am Ende des Zuchtzyklus;
ii) einen Palettierportal (3) für einen Stapel (17) von Behältern, um einen nachfolgenden Zyklus der Insektenproduktion zu starten;
iii) eine Handhabungskammer (4), die dazu ausgelegt ist, alle Übergangsoperationen für die Behälter zwischen den beiden Portalen durchzuführen;
wobei die Handhabungskammer (4) einen Behälterhandhabungsturm (5) umfasst, in dem vertikal angeordnet sind:
iv) ein Behälterhandhabungsmodul (6) zum Verschieben und Drehen der Behälter;
v) ein Modul (7) zum Aufnehmen von Materialien am Ende der Produktionszyklen, das unterhalb des Behälterhandhabungsmoduls (6) angeordnet ist;
vi) ein Füllmodul (8) mit Material für den Start des nächsten Insektenproduktionszyklus, das über dem Behälterhandhabungsmodul (6) angeordnet ist,
**dadurch gekennzeichnet, dass** im Handhabungssystem (1) der Empfangsportal (2) für einen ankommenden Behälterstapel am Ende eines Produktionszyklus und der Palettierportal (3) für einen abgehenden Stapel von Startmaterial für den nächsten Insektenproduktionszyklus auf beiden Seiten des Handhabungsturms (5) angeordnet sind.

2. Handhabungssystem (1) nach Anspruch 1, wobei das Behälterhandhabungsmodul (6) Schienen (11) umfasst, die zwischen dem Empfangsportal (2) und dem Palettierportal (3) angeordnet sind, wobei ein mobiler Wagen (12) auf diesen Schienen (11) montiert ist.

3. Handhabungssystem (1) nach Anspruch 2, bei dem der mobile Wagen (12) einen Drehpunkt (13) umfasst, der dazu ausgelegt ist, mindestens einen Behälter zu kippen und dessen Inhalt auszugießen.

4. Handhabungssystem (1) nach Anspruch 1, bei dem das Modul (7) zum Aufnehmen von Materialien am Ende des Produktionszyklus eine Sieb- und Rückgewinnungsvorrichtung (14) für Materialien am Ende des Zyklus umfasst, die dazu ausgelegt ist, die zurückzugewinnenden Komponenten aus dem Produktionsmaterial am Ende des Zyklus abzutrennen und diese Komponenten zu mindestens einem Punkt (15) zum Zurückgewinnen der abgetrennten Materialien zu befördern.

5. Handhabungssystem nach Anspruch 4, wobei die Sieb- und Rückgewinnungsvorrichtung (14) mindestens zwei Siebstufen umfasst.

6. Handhabungssystem nach einem der Ansprüche 1 bis 5, das ein Absaugsystem (16) umfasst, das so angeordnet ist, dass es die Larvenhäute und den Staub aus der Insektenproduktion zurückgewinnt.

7. Handhabungssystem nach einem der Ansprüche 1 bis 6, wobei der Empfangsportal (2) und der Palettierportal (3) einen Aufzug (9) umfassen, der so ausgelegt ist, dass er die Positionierung eines Behälters ermöglicht, der sich oben auf einem Stapel von Behältern am Handhabungsmodul (6) befindet.

## Claims

1. Handling system (1) for performing a plurality of transition operations between two production cycles with a plurality of bins (10) at the end of an insect rearing cycle in said bins, and preparing these bins for a subsequent insect rearing cycle, comprising:
i) a receiving gantry (2) for a stack (18) of bins at the end of the rearing cycle;
ii) a palletizing gantry (3) for a stack (17) of bins to start a subsequent cycle of insect production;
iii) a handling enclosure (4) designed to carry out all transition operations for the bins between the two gantries;
in which the handling enclosure (4) comprises a bin handling tower (5) in which the following are arranged vertically:
iv) a bin handling module (6) for moving the bins in translation and rotation;
v) a module (7) for receiving materials at the end of production cycles, arranged below the bin handling module (6);
vi) a filling module (8) with material for starting the next insect production cycle, arranged above the bin handling module (6),
**characterized in that** in the handling system (1), the receiving gantry (2) for an incoming stack of bins at the end of a production cycle and the palletizing gantry (3) for an outgoing stack of starter material for the next insect production cycle are arranged on either side of the handling tower (5).

2. Handling system (1) according to claim 1, in which the bin handling module (6) comprises rails (11) arranged between the receiving gantry (2) and the palletizing gantry (3), a mobile trolley (12) being mounted on said rails (11).

3. Handling system (1) according to claim 2, in which the mobile trolley (12) comprises a pivot (13) adapted to tilt at least one bin and pour out its contents.

4. Handling system (1) according to claim 1, in which the module (7) for receiving end-of-production cycle materials comprises a sieving and recovery device (14) for end-of-cycle material designed to separate the components to be recovered from the end-of-cycle production material and convey these components to at least one point (15) for recovering the separated materials.

5. Handling system according to claim 4, wherein the sieving and recovery device (14) comprises at least two sieving levels.

6. Handling system according to any of claims 1 to 5, comprising a suction system (16) arranged to recover the larval molts and dust resulting from insect production.

7. Handling system according to any of claims 1 to 6, wherein the bin receiving gantry (2) and the bin palletizing gantry (3) comprise an elevator (9) designed to allow the positioning of a bin located on top of a stack of bins at the handling module (6).
